# EUROPEAN PATENT APPLICATION

(11) **EP 4 327 798 A1**
(43) Date of publication of application: **28.02.2024**
(21) Application number: 22791582.4
(22) Date of filing: 31.03.2022
(51) Int. Cl.: A61K 6/30, A61K 6/60, A61K 6/70, A61K 6/79, A61K 6/831, A61K 6/889

(54) **DENTAL ADHESIVE COMPOSITION**

(30) Priority: 19.04.2021 JP 2021070438
(71) Applicant: Tokuyama Dental Corporation, Tokyo 110-0016 (JP)
(72) Inventor: MORIMOTO, Tetsuro, Tokyo 1100016 (JP); FUKUDOME, Keishi, Tokyo 1100016 (JP)
(74) Representative: Markfort, Iris-Anne Lucie
(86) International application number: PCT/JP2022/016842
(87) International publication number: WO 2022/224807

(57) **Abstract**

[Object] To provide a dental adhesive composition that has high adhesiveness even to a silica ceramic restoration by containing a silane coupling material, can be stably preserved for a long period of time even with presence of both a silica filler and the silane coupling material, and is suitable as dental cement.

[Solving Means] A dental adhesive composition includes: 100 parts by mass of a polymerizable monomer component (A) formed of an acidic group-containing polymerizable monomer and an acidic group-free polymerizable monomer; 50 to 1000 parts by mass of a silica filler (B); and 0.1 to 20 parts by mass of a silane coupling material (C), in which 97 mass% or more of the silane coupling material (C) is an α-branched alkoxysilane silane coupling material formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position.

## Description

### Technical Field

The present invention relates to a dental adhesive composition.

### Background Art

In dental treatment, an adhesive curable composition that contains a polymerizable monomer component containing a polymerizable monomer having an acidic group (hereinafter, referred to also as an "acidic monomer".), a filler, and a polymerization initiator is widely used. The filler is mixed for various purposes such as adjustment of the viscosity of the paste and improvement in aesthetics of the cured product, and a silica filler is often used as the filler because the shape, size, and the like of particles can be easily controlled (see Patent Literatures 1 to 3).

One of such adhesive curable compositions is dental cement (particularly, dental resin cement). In the case where a tooth substance damaged by caries or the like is restored with a prosthesis formed of various metals, various ceramics, or the like, resin cement is generally used to bond the prosthesis and the tooth substance.

Since the dental (resin) cement, particularly self-adhesive resin cement, contains an acidic monomer, it has adhesiveness to a tooth substance, metal, and zirconia and can also be mixed with a silane coupling material(γ-methacryloxypropyl trimethoxysilane, γ-methacryloxypropyl triethoxysilane, or the like) to enhance the adhesiveness to a restoration formed of silica ceramics such as porcelain. However, in the case where the silane coupling material is mixed, it is necessary to separately preserve the silane coupling material and an acid component such as a polymerizable monomer having an acidic group separately in two agents such that they do not coexist and mix them at the time of use. Even in such a usage form, there remains a problem of preservation stability. That is, the filler is mixed in each of the two agents described above in order to prevent a difference in the paste properties between the respective agents from occurring from the viewpoint of operability during use, but using a silica filler as the filler causes a problem that the silane coupling material is deactivated due to the influence of the surface acid sites of the silica filler during long-term preservation.

Under such circumstances, some types of dental cement containing a silane coupling material have also been developed. For example, Patent Literature 4 discloses a "two-paste type dental curable composition including: a first agent that contains a polymerizable monomer (a) having an acidic group, a polymerizable monomer (b) having no acidic group, a polymerization initiator (c), and a filler (d) and further contains, as necessary, a photopolymerization initiator (h), a polymerization inhibitor, a coloring agent, a fluorescent agent, an antibacterial substance, an ultraviolet absorber, a dye, and a pigment, the content of components other than the components described above being less than 1.0 weight!, the polymerization initiator (c) being one or more selected from the group consisting of an organic peroxide, an inorganic peroxide, and a transition metal complex; and a second agent that contains a polymerizable monomer (b) having no acidic group, at least one basic filler (e) selected from the group consisting of a basic glass filler and alumina, a polymerization accelerator (f), and a silane coupling agent (g) represented by the following general formula [I]. 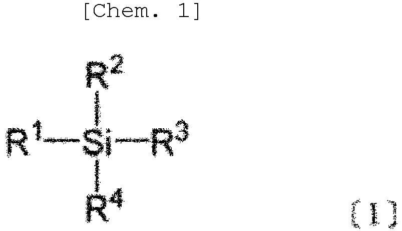 (In the formula, R¹ represents an organic residue having at least one functional group selected from the group consisting of a (meth)acryloyl group, a vinyl group, and an epoxy group, R² represents a hydroxy group, an alkyl group having 1 to 5 carbon atoms, or an alkoxy group having 1 to 5 carbon atoms, R³ and R⁴ each represent a hydroxy group or an alkoxy group having 1 to 5 carbon atoms, and at least one of R² to R⁴ is an alkoxy group having 2 to 5 carbon atoms.)

### Citation List

### Patent Literature

Patent Literature 1: Japanese Patent No. 6854155
Patent Literature 2: Japanese Patent Application Laid-open No. 1988-51308
Patent Literature 3: WO2009/084586 pamphlet
Patent Literature 4: Japanese Patent No. 6346086

### Disclosure of Invention

### Technical Problem

In the two-paste type dental curable composition described in Patent Literature 4, it is necessary to use the basic filler (e) such as barium glass as the filler in the second agent containing a silane coupling material and it is difficult to match, for example, in the case where a silica filler is used as the filler (d) of the first agent, the paste properties of both agents (even if the silica filler is additionally mixed within a certain range in the second agent).

In this regard, it is an object of the present invention to provide a dental adhesive composition that can be suitably used as dental cement, particularly dental resin cement, has high adhesiveness even to a silica ceramic restoration by containing a silane coupling material, and can be stably preserved for a long period of time even with presence of both a silica filler and the silane coupling material.

### Solution to Problem

The present invention is intended to solve the problem described above. A first embodiment of the present invention is a dental adhesive composition, including: 100 parts by mass of a polymerizable monomer component (A) in which 1 to 30 parts by mass is an acidic group-containing polymerizable monomer (a1) and the remainder is an acidic group-free polymerizable monomer (a2); 50 to 1000 parts by mass of a silica filler (B); and 0.1 to 20 parts by mass of a silane coupling material (C), the dental adhesive composition being characterized in that
97 mass% or more of the silane coupling material (C) is an α-branched alkoxysilane silane coupling material (c1) formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position.

It is favorable that the dental adhesive composition according to the embodiment (hereinafter, referred to also as the "dental adhesive composition according to the present invention".) further contains a polymerization initiator (D) and further contains 1.5 to 300 parts by mass of water (E) with respect to 100 parts by mass of the silane coupling material (C).

A second embodiment of the present invention is a two-paste type dental adhesive composition for preparing the dental adhesive composition according to the present invention by mixing a paste-like first agent and a paste-like second agent, comprising: the first agent and the second agent, which are separately packaged, the two-paste type dental adhesive composition being characterized in that the first agent contains the acidic group-containing polymerizable monomer (a1), the acidic group-free polymerizable monomer (a2), and the silica filler (B) and does not contain the silane coupling material (C), the second agent contains the acidic group-free polymerizable monomer (a2), the silica filler (B), and the silane coupling material (C) and does not contain the acidic group-containing polymerizable monomer (a1), and in a case where the dental adhesive composition contains components other than the acidic group-containing polymerizable monomer (a1), the acidic group-free polymerizable monomer (a2), the silica filler(B), and the silane coupling material (C), the components are contained in the first agent and/or the second agent.

In the two-paste type dental adhesive composition according to the embodiment (hereinafter, referred to also as the "two-paste type dental adhesive composition according to the present invention".), it is favorable that the first agent and/or the second agent contains 1.5 to 300 parts by mass of water (D) with respect to 100 parts by mass of the silane coupling material (C), and it is particularly favorable that 3 to 12 parts by mass of the α-branched alkoxysilane silane coupling material (c1) is contained with respect to 100 parts by mass of the polymerizable monomer component (A), the first agent contains 1.8 to 12 parts by mass of water (D) with respect to 100 parts by mass of the α-branched alkoxysilane silane coupling material (c1), and the second agent does not contain water(D) or contains 10 parts by mass or less of water (D).

A third embodiment of the present invention is dental cement, including: the dental adhesive composition according to the present invention. A fourth embodiment of the present invention is two-paste type dental cement, including: the two-paste type dental adhesive composition according to the present invention.

### Advantageous Effects of Invention

Since the dental adhesive composition according to the present invention contains an acid monomer and a silane coupling material, it has excellent features that it has high adhesiveness not only to materials such as a tooth substance, metal, and zirconia but also to silica ceramics and can be stably preserved for a long period of time by being divided into two agents, i.e., the two-paste type dental adhesive composition according to the present invention. Further, when dividing into two agents, since the silane coupling material (C) is stable even if it coexists with a silica filler, the same type of filler can be mixed in both agents and the paste properties of the agents can be easily matched.

Therefore, the dental adhesive composition according to the present invention and the two-paste type dental adhesive composition according to the present invention can be suitably used as dental (resin) cement.

### Mode(s) for Carrying Out the Invention

The excellent effect as described above can be achieved by using, as a silane coupling material (hereinafter, referred to also as an "SC material".), an "a-branched alkoxysilane" (referred to also as "branched RO".) silane coupling material (SC material) (c1) "formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position and making the SC material substantially not contain another silane coupling material, specifically, making 97 mass% or more, favorably 99 mass% or more of the SC material to be used be the α-branched alkoxysilane SC material.

Although details of why the effect is achieved by using the α-branched alkoxysilane SC material (c1) are unknown, the present inventors presume that the effect is achieved due to the following reasons because the acceleration effect is observed in the case where water is contained. That is, it is presumed that since the α-branched alkoxysilane SC material has a bulky structure because the alkoxy, which is a leaving group during hydrolysis, has a branch at an α position, the effects of the acid sites present on the surface of the filler (solid) are blocked to suppress hydrolyzed and condensation and high preservation stability is exhibited. Meanwhile, it is conceivable that since the access of the acid component dissolved in a liquid is not hindered, hydrolysis occurs due to the action of the acid component such as an acid monomer during use, the α-position branched alkoxy group leaves to form a silanol group, this silanol group bonds with a hydroxy group on the surface of the silica filler through dehydration condensation, and thus, high adhesiveness to silica ceramics is exhibited.

Note that in Patent Literature 4, specific examples of the silane coupling material (g) represented by the formula [I]include vinyl triethoxysilane, vinyl tris(β-methoxyethoxy)silane, vinyl tripropoxysilane, vinyl tributoxysilane, γ-methacryloxypropyl triethoxysilane, γ-methacryloxypropyl tris(β-methoxyethoxy)silane, 6-(meth)acryloxyhexyl triethoxysilane, κ-methacryloxydecyl triethoxysilane, and 11-(meth)acryloyloxy undecyl triethoxysilane, and Patent Literature 4 does not describe the α-branched alkoxysilane SC material.

The present invention will be described below in detail. Note that in this specification, unless otherwise specified, the notation "x to y" using numerical values x and y means "x or more and y or less". In such notation, when only the numerical value y is given a unit, the unit is also applied to the numerical value x. Further, in this specification, the term "(meth)acrylic" means both "acrylic" and "methacrylic". Similarly, the term "(meth)acrylate" means both "acrylate" and "methacrylate" and the term "(meth)acryloyl" means both "acryloyl" and "methacryloyl".

### 1. Regarding components of dental adhesive composition according to present invention

### 1-1. Polymerizable monomer component (A)

The polymerizable monomer component (A) in the dental adhesive composition according to the present invention includes an acidic group-containing polymerizable monomer (acidic monomer) (a1) and an acidic group-free polymerizable monomer (hereinafter, referred to also as "non-acidic monomer".) (a2). From the viewpoint of activation of the α-branched alkoxysilane SC material and mechanical strength of the cured body, the amount of the acidic monomer (a1) in 100 parts by mass of the total amount of the polymerizable monomer (A) needs to be 1 to 30 parts by mass, is favorably 3 to 20 parts by mass, and particularly favorably 5 to 10 parts by mass. Note that the amount of the remainder after removing the amount of the acidic monomer (a1) is the amount of the acidic group-free polymerizable monomer (a2).

### 1-1-1. Acidic monomer (a1)

In the dental adhesive composition according to the present invention, the acidic monomer (a1) means a polymerizable monomer having an acidic group such as a carboxyl group, an acid anhydride group thereof, and a phosphonooxy group in the molecule, not only functions as an adhesive component by itself, but also has a function of reacting with and activating the α-branched alkoxysilane SC material (C). As the acidic monomer (a1), acidic monomers used in existing dental adhesive compositions can be used without particular limitation, and (meth)acrylate having an acidic group in the molecule is suitably used among them from the viewpoint of polymerizability.

Examples of the acidic monomer that can be suitably used include bis(6-(meth)acryloyloxyhexyl)hydrogenphosphate, bis(10-(meth)acryloyloxydecyl)hydrogenphosphate, bis{2-(meth)acryloyloxyethyl}hydrogenphosphate, bis{2-(meth)acryloyloxy-(1-hydroxymethyl)ethyl}hydrogenphosphate, pentaerythritol tri(meth)acrylatehydrogenphosphate, and dipentaerythritol penta(meth)acrylatehydrogenphosphate. Among them, from the viewpoint of availability, 2-(meth)acryloyloxy dihydrogenphosphate, bis{2-(meth)acryloyloxyethyl}hydrogenphosphate, 10-(meth)acryloyloxydecyl dihydrogenphosphate are favorable. Note that these acidic monomers may be used alone or two or more of them may be used in combination.

### 1-1-2. Non-acidic monomer (a2)

The non-acidic monomer (a2) is not particularly limited as long as it has at least one radically polymerizable unsaturated group in the molecule and does not have an acidic group. From the viewpoint of polymerizability and safety to the living body, (meth)acrylate is suitably used. Examples of the acidic group-free polymerizable monomer that can be suitably used include methyl(meth)acrylate, ethylene glycol di(meth)acrylate, triethylene glycol di(meth)acrylate, propylene glycol di(meth)acrylate, 2,2-bis[4-(3-methacryloyloxy)-2-hydroxypropoxyphenyl]propane, 2,2-bis(methacryloyloxypolyethoxyphenyl)propane, 2-hydroxyethylmethacrylate, 2,2,4-trimethylhexamethylene bis(2-carbamoyloxyethyl)dimethacrylate, and 1,10-decanediol di(meth)acrylate. These non-acidic monomers may be used alone or two or more of them may be used in combination.

Note that in the case where the cured body of the dental adhesive composition according to the present invention is required to have higher mechanical strength, it is favorable to use, as the non-acidic monomer, a di-, tri-, or tetra-functional radically polymerizable monomer having a plurality of radically polymerizable groups.

### 1-2. Silica filler (B)

In the dental adhesive composition according to the present invention, the silica filler (B) has the function of improving the mechanical strength of the composition and activating the α-branched alkoxysilane SC material (C). As the silica filler (B), silica fillers used in existing dental materials or the like can be used without particular limitation. Further, only one type of silica filler may be used, or two or more types may be used in combination.

The silica filler is inorganic particles containing silica as a main component. Containing silica as a main component means that the filler contains 60 mass% or more of the silica component, and 70 mass% or more of the silica component is favorably contained.

The shape of the silica filler is not particularly limited. The silica filler may be amorphous particles or particles having a spherical shape. The average particle size of the silica filler is not particularly limited, but is favorably approximately 0.01 µm to 100 µm, and more favorably approximately 0.1 µm to 50 µm.

Examples of the silica filler that is particularly suitably used include a composite oxide containing silicon as a constituent element, such as silica-titania and silica-zirconia; and a clay mineral or a silicate containing silicon as a constituent element, such as talc, montmorillonite, zeolite, and calcium silicate. These silica fillers are subjected to surface treatment with a surface treatment agent such as a silane coupling material in order to improve compatibility with the polymerizable monomer and improve the mechanical strength and water resistance of the obtained cured body and then used in some cases. The silica filler is favorable because it has excellent chemical stability and can be easily surface-treated with a silane coupling material or the like, and a composite oxide containing silicon as a constituent element, such as silica-titania and silica-zirconia, is particularly favorable.

The content of the silica filler (B) is 50 to 1000 parts by mass, favorably 100 to 300 parts by mass, with respect to 100 parts by mass of the polymerizable monomer (A) contained in the dental material from the viewpoint of kneadability and the mechanical strength of the cured body.

### 1-3. Silane coupling material (C)

The dental adhesive composition according to the present invention contains 0.1 to 21 parts by mass, favorably 1 to 18 parts by mass, more favorably 3 to 12.5 parts by mass, of the silane coupling material (SC material) (C) with respect to 100 parts by mass of the polymerizable monomer component (A) in order to improve adhesiveness to a silica ceramic restoration. Then, in order to allow the dental adhesive composition to be stably preserved even if it coexists with a silica filler, 97 mass% or more of the SC material (C) is the α-branched alkoxysilane SC material (c1). When the content ratio of the α-branched alkoxysilane SC material (c1) is less than 97 mass%, it is difficult to achieve high preservation stability. From the viewpoint of the effect, the proportion of the α-branched alkoxysilane SC material (c1) in the SC material is favorably 99 mass% or more. The α-branched alkoxysilane SC material (c1) will be described below in detail.

### 1-3-1. α-branched alkoxysilane SC material (c1)

The α-branched alkoxysilane SC material (c1) means a silane coupling material formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position.

Suitable examples of the radically polymerizable functional group of the monovalent radically polymerizable organic residue include a (meth)acryloyl group, a (meth)acryloyloxy group, a vinyl group, and a (meth)acrylamide group. As the monovalent radically polymerizable organic residue, a vinyl group or a group in which a hydrogen atom bonded to the terminal carbon atom of an alkyl group having 1 to 20 carbon atoms, particularly a linear alkyl group having 3 to 10 carbon atoms, is substituted with a radically polymerizable functional group is favorable. Examples of the group suitable as the monovalent radically polymerizable organic residue include a vinyl group, a 3-(meth)acryloxypropyl group, a 5-(meth)acryloxypentyl group, a 7-methacryloxyheptyl group, and 9-(meth)acryloxynonyl group.

Examples of the branched alkoxy group having 3 to 5 carbon atoms and a branch at an α position include an isopropoxy group, a cyclopropoxy group, a sec-butoxy group, a tert-butoxy group, and a cyclopentoxy group.

In the α-branched alkoxysilane SC material (c1), two of the four groups bonded to a silicon atom only need to be the monovalent radically polymerizable organic residue and the branched alkoxy group having 3 to 5 carbon atoms and a branch at an α position. The α-branched alkoxysilane SC material (c1) may have a group (referred to also as a "different group") other than these groups. In the case where the α-branched alkoxysilane SC material (c1) has the different group, the different group is favorably a hydrocarbon group having 1 to 6 carbon atoms such as a methyl group, an ethyl group, a propyl group, a butyl group, a pentyl group, a hexyl group, and a phenyl group.

When the "monovalent radically polymerizable organic residue" bonded to a silicon atom is defined as R₁, the number of bonds thereof is defined as a; the "branched alkoxy group having 3 to 5 carbon atoms" bonded to a silicon atom is defined as R₂, the number of bonds thereof is defined as b; the "different group" that can be bonded to a silicon atom is defined as R₃, and the number of bonds thereof is defined as c, an organosilicon compound that is the α-branched alkoxysilane SC material (c1) can be represented by the general formula: Si(R₁)ₐ(R₂)_{b}(R₃)_{c}. However, in the formula, a is an integer of 1 to 3, b is an integer of 1 to 3, c is an integer of 0 to 2, and the relationship of a + b + c = 4 is always satisfied.

Examples of the α-branched alkoxysilane SC material (organosilicon compound) that can be suitably used include vinyl triisopropoxysilane, vinyl diisopropoxymethylsilane, vinylisopropoxy dimethylsilane, 1-(meth)acryloxymethyl triisopropoxysilane, 2-(meth)acryloxyethyl triisopropoxysilane, 3-(meth)acryloxypropyl triisopropoxysilane, 5-(meth)acryloxypentyl triisopropoxysilane, 7-(meth)acryloxyheptyl triisopropoxysilane, 9-(meth)acryloxynonyl triisopropoxysilane, 11-(meth)acryloxyundenyl triisopropoxysilane, 3-(meth)acryloxypropyl diisopropoxymethylsilane, and 3-(meth)acryloxypropyl tritert-butoxysilane. Among them, 3-(meth)acryloxypropyl triisopropoxysilane and 3-(meth)acryloxypropyl tritert-butoxysilane are favorably used from the viewpoint of adhesiveness and availability. Note that these α-branched alkoxysilane SC materials (organosilicon compounds) may be used alone or two or more of them may be used in combination.

The mixing amount of the α-branched alkoxysilane SC material (c1) is 0.1 to 20 parts by mass, favorably 1 to 17 parts by mass, more favorably 3 to 12 parts by mass, with respect to 100 parts by mass of the polymerizable monomer (A) contained in the dental material from the viewpoint of adhesiveness and mechanical strength.

### 1-3-2. SC material (c2) other than α-branched alkoxysilane SC material

As the SC material (c2) other than the α-branched alkoxysilane SC material, which may be contained in the SC material to be mixed in the dental adhesive composition according to the present invention as long as the content thereof is 3 mass% or less, favorably 1 mass% or less, the SC material formed of an organosilicon compound such as γ-methacryloxypropyl triethoxysilane and γ-methacryloxypropyl trimethoxysilane, which is mixed in existing dental adhesive compositions, can be used without particular limitation as long as such a condition is satisfied.

### 1-4. Other arbitrary components

### 1-4-1. Water (D)

In the dental adhesive composition according to the present invention, 1.5 to 300 parts by mass, particularly favorably 1.5 to 100 parts by mass, most favorably 1.8 to 12 parts by mass, of the water (D) is contained with respect to 100 parts by mass of the α-branched alkoxysilane SC material (c1) in order to promote the adhesion and increase the adhesive strength of the α-branched alkoxysilane SC material and the silica ceramic restoration by hydrolyzing the α-branched alkoxysilane SC material.

### 1-4-2. Non-silica filler (E)

In the dental adhesive composition according to the present invention, a filler (non-silica filler) other than the silica filler, such as fluoroaluminosilicate glass, lanthanum glass, barium glass, and strontium glass, may be mixed. The mixing amount of the non-silica filler is favorably 10 parts by mass or less with respect to 100 parts by mass of the silica filler (B) from the viewpoint of activating the α-branched alkoxysilane SC material (c1).

### 1-4-3. Polymerization initiator (F)

The polymerization initiator (F) has the function of polymerizing and curing the composition. Examples of the polymerization initiator (F) include a photopolymerization initiator, a chemical polymerization initiator, and a thermal polymerization initiator. In dental adhesives and dental cement, a photopolymerization initiator and a chemical polymerization initiator are particularly used from the viewpoint of operability in the oral cavity. As the initiators, known ones can be used without particular limitation. These polymerization initiators may be used alone or two or more of them may be used in combination.

Representative examples of the photopolymerization initiator include α-diketones, ketals, thioxanthones, (bis)acylphosphineoxides, and α-aminoacetophenones. Further, in general, α-diketones and thioxanthones act as polymerization initiators when used in combination with amines. These polymerization initiators may be used alone or two or more of them may be mixed in combination.

Examples of the amines include an aromatic amine and an aliphatic amine. These may be used alone or two or more of them may be mixed in combination.

Examples of the chemical polymerization initiator include a chemical polymerization initiator in which an oxidizing agent, a reducing agent, and, if necessary, a transition metal compound are combined.

Examples of the oxidizing agent include an organic peroxide and an inorganic peroxide. These oxidizing agents may be used alone or two or more of them may be mixed in combination.

Representative examples of the organic peroxide include a hydroperoxide, a peroxyester, a ketone peroxide, a peroxyketal, a dialkylperoxide, a diacylperoxide, and a peroxydicarbonate. These organic peroxides may be used alone or two or more of them may be used in combination.

Examples of the inorganic peroxide include a peroxydisulfate and a peroxodiphosphate. Among them, the inorganic peroxides may be used alone or two or more of them may be used in combination.

Examples of the reducing agent include amines, an aromatic sulfinate, a thiourea compound, and an aryl borate compound. The reducing agents may be used alone or two or more of them may be mixed in combination.

Examples of the transition metal compound include a 4th period transition metal compound.

In the 4th period transition metal compound, the 4th period transition metal is a metal element of Groups 3 to 12 of the 4th period of the periodic table, favorably vanadium(V) or copper (Cu). Each of these transition metal elements can have a plurality of valences, but those with valences that can exist stably, e.g., a compound of V (III to V) or Cu (I, II) is used as a chemical polymerization initiator in combination with an organic peroxide and a reducing agent.

Among them, a +IV-valent and/or +V-valent vanadium compound or +I-valent and/or +II-valent copper compound is more favorable. The +IV-valent vanadium compound or +II-valent copper compound are the most favorable. These can be used alone or two or more of them can be used in combination.

### 2. Regarding usage form of dental adhesive composition according to present invention

As described above, even in the case where substantially only the α-branched alkoxysilane SC material (c1) is used as a silane coupling material, favorable reservation stability cannot be achieved when it coexists with an acidic monomer and is preserved. For this reason, the respective components constituting the dental adhesive composition according to the present invention, particularly the α-branched alkoxysilane SC material (c1) and the acidic monomer (a1), are a combination of two or more partial compositions in which they are maintained in a state where they cannot come into chemical contact with each other, and are favorably mixed and used at the time of use.

Note that in this specification, the "state where they cannot come into chemical contact with each other" means (1) a case where one composition and another composition are in a "state where they cannot come into physical contact with each other", or (2) a case where although one composition and another composition can come into physical contact with each other, unidirectional or bidirectional molecular diffusion does not occur between one composition and another composition. Specific examples of the latter (2) include a state where one composition and another composition are in contact with each other in a completely solidified state in the temperature environment during preservation of the composition.

Further, in this specification, the "state where they cannot come into physical contact with each other" means a state where one composition and another composition are away from each other by an inhibitor that inhibits molecular diffusion between the two. As the inhibitor, a solid member such as a resin, glass, metal, and ceramics, which are suitably used as materials of containers and bags, is generally used. Typical examples of the "state where they cannot come into physical contact with each other" include a state where one type of composition is preserved while being hermetically sealed in a container blocking from outside air and outside light.

In the case where the respective components constituting the dental adhesive composition according to the present invention are a combination of two or more partial compositions that are kept in a state where they cannot come into chemical contact with each other, it is favorable to distribute the components to the respective partial compositions such that the components that react with each other when mixed are distributed to the respective partial compositions from the viewpoint of preservation stability.

Note that from the practical point of view, the dental adhesive composition according to the present invention favorably includes a combination of two partial compositions, i.e., the two-paste type dental adhesive composition according to the present invention.

The two-paste type dental adhesive composition according to the present invention will be described below.

### 3. Two-paste type dental adhesive composition according to present invention

The two-paste type dental adhesive composition according to the present invention is a two-paste type dental adhesive composition for preparing the dental adhesive composition according to the present invention by mixing a paste-like first agent and a paste-like second agent, including: the first agent and the second agent, which are separately packaged.

Then, the first agent includes a first partial composition that contains the acidic group-containing polymerizable monomer (a1), the acidic group-free polymerizable monomer (a2), and the silica filler (B) and does not contain the silane coupling material (C).

Further, the second agent includes a second partial composition that contains the acidic group-free polymerizable monomer (a2), the silica filler (B), and the silane coupling material (C) and does not contain the acidic group-containing polymerizable monomer (a1).

Note that in the case where the dental adhesive composition according to the present invention contains other arbitrary components, i.e., components other than the acidic group-containing polymerizable monomer (a1), the acidic group-free polymerizable monomer (a2), the silica filler(B), and the silane coupling material (C), the components are appropriately contained in the first agent and/or the second agent (the first partial composition and/or the second partial composition) in consideration of preservation stability and the like.

At this time, in the case where the water (D) is contained as another arbitrary component, it is favorable to mix the water (D) in the first agent. It is favorable to mix 1.8 to 12 parts by mass of the water (D) in the first agent with respect to 100 parts by mass of the α-branched alkoxysilane SC material (c1) because adhesiveness to silica ceramics increases and high adhesiveness can be maintained even after long-term preservation. Note that it is favorable not to mix water or mix 10 parts by mass or less of water even if it is mixed, because the adhesive strength tends to decrease when a large amount of water is mixed in the second agent.

Further, another filler (E) is contained as another arbitrary component, it may be mixed in only one of the first agent and the second agent (the first partial composition and the second partial composition) or may be mixed separately.

Further, in the case where the polymerization initiator (F) is mixed as another arbitrary component, care must be taken that the polymerizable monomer component is not polymerized during preservation. Specifically, in the case where the polymerization initiator (F) is a photopolymerization initiator, there is no problem even if it coexists with the monomer component (A) as long as it is not activated by blocking light, for example. However, care must be taken when using a chemical polymerization initiator. Since the non-acidic monomer (a2) is contained in both the first agent and the second agent, it is necessary to use a chemical polymerization initiator containing a plurality of components, each of which does not have polymerization activity with respect to the monomer component (A), and separate these components such they are not polymerized in the first agent and the second agent. For example, in the case where a chemical polymerization initiator that exhibits polymerization activity through the action of an oxidizing agent and a reducing agent with the presence of a transition metal compound is used as the chemical polymerization initiator, it is necessary to mix the transition metal compound only in the first agent and the reducing agent only in the second agent from the viewpoint of preservation stability. At this time, it is favorable to mix the oxidizing agent only in the first agent, but the oxidizing agent can be mixed in the second agent in accordance with the type of oxidizing agent.

Since the two-paste type dental adhesive composition according to the present invention is for preparing the dental adhesive composition according to the present invention by mixing the first partial composition and the second partial composition, the composition of the dental adhesive composition according to the present invention to be prepared (which is a mixture of both the partial compositions) is determined by the composition of both the partial compositions and the mixing ratio. That is, in the composition of the dental adhesive composition according to the present invention to be prepared, the mixing amount (parts by mass) of the component to be mixed only in one of the first partial composition and the second partial composition (referred to also as the non-common component.) is the mixing amount (parts by mass) of the non-common component contained in the first partial composition or the second partial composition used for mixing, and the mixing amount of the component to be mixed in both the first partial composition and the second partial composition (referred to also as the common component.) is the sum of the mixing amounts (parts by mass) of the common component contained in the first partial composition and the second partial composition used for mixing. Therefore, when distributing the common component in the first partial composition and the second partial composition, it is necessary to distribute the common component by assuming the mixing ratio. The distribution of the common component, specifically, the non-acidic monomer (a2), the silica filler (B), and other arbitrary components to be mixed in both the partial compositions may be appropriately determined in consideration of the ease of handling of each partial composition after the distribution, the ease of packaging when the composition is made into a kit, and the like. At this time, since it may be difficult to handle and product packaging may be difficult in the case where the masses or volumes of the first partial composition and the second partial composition to be mixed are too different from each other, the distribution of the common component is favorably made such that the mixing ratio of the first partial composition and the second partial composition to be used to constitute the composition is within a range of the first partial composition: the second partial composition = 1/5 to 5/1 in mass ratio or volume ratio and then the mixing amount of the common component satisfies the (compositional) condition of the dental adhesive composition according to the present invention on the basis of the mixing amount of the composition contained after mixing. Note that the mixing ratio is more favorably in the range of 1/3 to 3/1 and particularly favorably 1/1.

### (Example)

Although the present invention will be described below with reference to Examples, the present invention is not limited only to the following Example.

### 1. Various raw materials

First, raw materials used to prepare a dental adhesive composition in Examples and Comparative Examples, which are respective components, will be described.

### (1) Polymerizable monomer (monomer) (A) component

### (1-1) Acidic monomer (a1)

MDP; 10-methacryloxydecyl dihydrogenphosphate
SPM; 1:2 mixture of 2-methacryloyloxy dihydrogenphosphate and bis{2-methacryloyloxyethyl}hydrogenphosphate.

### (1-2) Non-acidic monomer (a2)

BisGMA; 2,2-bis[4-(3-methacryloyloxy)-2-hydroxypropoxyphenyl]propane
D-2.6E; 2,2-bis(methacryloyloxypolyethoxyphenyl)propane
3G; triethylene glycol dimethacrylate
UDMA; 2,2,4-trimethylhexamethylene bis(2-carbamoyloxyethyl)dimethacrylate
HEMA; 2-hydroxyethylmethacrylate.

### (2) Filler component

### (2-1) Silica filler (B)

The following silica-zirconia filler was synthesized using tetraethylsilicate and tetrabutyl zirconate in accordance with the method disclosed in Japanese Patent No. 3277502.
F1; Silica-zirconia filler with an average particle size of 3 µm (silica content of 80 mass%)
F2; Silica-zirconia filler with an average particle size of 0.2 µm (silica content of 80mass%).

### (2-2) Non-silica filler (E)

G1: Glass filler with an average particle size of 3 µm (GM27884, manufactured by SCHOTT AG, silica content of 55 mass%)
G2: Glass filler with an average particle size of 0.4 µm (GM27884, manufactured by SCHOTT AG, silica content of 55 mass%)
G3: Glass filler with an average particle size of 3 µm (G018-117, manufactured by SCHOTT AG, silica content of 50 mass%)
G4: Glass filler with an average particle size of 0.4 µm (G018-117, manufactured by SCHOTT AG, silica content of 50 mass%)
G5: Glass filler with an average particle size of 3 µm (pulverized V-124-1125, manufactured by ESSTECH Inc., silica content of 57%)
G6: Glass filler with an average particle size of 0.4 µm (pulverized V-124-1125, manufactured by ESSTECH Inc., silica content of 57%).

### (3) Silane coupling material (SC material) (C) component

### (3-1) α-branched alkoxysilane (branched RO) SC material (c1)

MPTiPS; 3-methacryloxypropyl triisopropoxysilane
MPDiPMS; 3-methacryloxypropyl diisopropoxymethylsilane
MPTtBS; 3-methacryloxypropyl tritert-butoxysilane
MHTiPS; 7-methacryloxyheptyl triisopropoxysilane.

### (3-2) SC material other than α-branched RO (other SC materials) (c2)

MPTES; γ-methacryloxypropyl triethoxysilane
MPTMS; γ-methacryloxypropyl trimethoxysilane.

### (4) Polymerization initiator (F) component

CuA; Copper (II) acetate monohydrate
CuAA; Copper (II) acetylacetone
BMOV; Bis(maltolate)oxovanadium (IV)
VOAA; Vanadium oxide acetylacetonate (IV)
BPT; t-butylperoxy-3,5,5-trimethylhexanoate
TMBPH; 1,1,3,3-tetramethylbutylhydroperoxide
CHP; Cumene hydroperoxide
CQ; Camphorquinone
PhBNa; Sodium salt of tetraphenylboron
PhBTEOA; Triethanolamine salt of tetraphenylboron
AcTU; Acetylthiourea
BzTU; Benzoylthiourea
DMBE; 4-dimethylaminoethyl benzoate.

### (5) Monomer composition

First agent monomer compositions I-1 to I-15 and second agent monomer compositions II-1 to II-6 having the compositions shown in Table 1 and Table 2 were prepared and used with the monomer component (A) and the polymerization initiator component (F) to be used in preparing the first agent and the second agent in each of the Examples and the Comparative Examples as monomer compositions. Note that the numbers in parentheses in the Tables represent parts by mass and the "upward arrow" means the same as above. Further, the notation of X(x)/Y(y) means a mixture of X: x parts by mass and Y: y parts by mass.

**[Table 1]**

| First agent monomer composition | | | |
|---|---|---|---|
| Symbol | Monomer (A) | | Polymerization initiator (F) |
| | Acidic monomer (a1) | Non-acidic monomer (a2) | |
| I -1 | MDP(10) | HEMA(10) / 3G(20) / D-2.6E(10) | BPT(2) / CuA(0.02) |
| I -2 | MOP(1) | HEMA(19) / 3G(20) / D-2.6E(10) | ↑ |
| I -3 | MDP(3) | HEMA(17) / 3G(20) / D-2.6E(10) | ↑ |
| I -4 | MDP(5) | HEMA(15) / 3G(20) / D-2.6E(10) | ↑ |
| I -5 | MDP(20) | HEMA(10) / 3G(10) / D-2.6E(10) | ↑ |
| I -6 | MDP(30) | HEMA(10) / D-2.6E(10) | ↑ |
| I -7 | SPM(10) | ↑ | ↑ |
| I -8 | ↑ | ↑ | BPT(2) / CuAA(0.02) |
| I -9 | ↑ | ↑ | BMOV(0.01) |
| I -10 | ↑ | ↑ | VOAA(0.01) |
| I -11 | ↑ | ↑ | CQ(2) |
| I -12 | ↑ | HEMA(10) / 3G(10) / UDMA(20) | BPT(2) / CuA(0.02) |
| I -13 | ↑ | HEMA(10) / 3G(20) / D-2.6E(10) | ↑ |
| I -14 | MDP(0.5) | HEMA(19.5) / 3G(20) / D-2.6E(10) | BPT(2) / CuA(0.02) |
| I -15 | MDP(40) | HEMA(10) | ↑ |

**[Table 2]**

| Second agent monomer composition | | |
|---|---|---|
| Symbol | Monomer (A) | Polymerization initiator (F) |
| | Non-acidic monomer (a2) | |
| II -1 | BisGMA(30) / 3G(20) | AcTU(1) / PhBNa(2) |
| II -2 | ↑ | BzTU(1) / PhBTEOA(2) |
| II -3 | ↑ | TMBPH(1) / PhBNa(2) |
| II -4 | ↑ | CHP(1) / PhBTEOA(2) |
| II -5 | ↑ | DMBE(2) |
| II -6 | UDMA(30) / 3G(20) | AcTU(1) / PhBNa(2) |

### 2. Examples and Comparative Examples

### Example 1

### (1) Preparation of two-paste type dental adhesive composition (first agent and second agent)

The first agent monomer composition I-1 and the second agent monomer composition II-1 were used to prepare the first partial composition and the second partial composition having the following composition (each of which is a paste-like composition containing a monomer component and a filler component), which were respectively used as the first agent and the second agent.

### [Composition of first agent (first partial composition)] ·Monomer (A) component

Acidic monomer (a1): 10 parts by mass of MDP
Non-acidic monomer (a2): Mixture of 10 parts by mass of HEMA, 10 parts by mass of D-2.6E, and 20 parts by mass of 3G

### ·Filler component

Silica filler (B): 54 parts by mass of F1 and 36 parts by mass of F2

### ·Polymerization initiator (F) component

2 parts by mass of BPT and 0.02 parts by mass of CuA

### [Composition of second agent (second partial composition)] ·Monomer (A) component

Non-acidic monomer (a2): Mixture of 30 parts by mass of BisGMA and 20 parts by mass of 3G

### ·Filler component

Silica filler (B): Mixture of 54 parts by mass of F1 and 36 parts by mass of F2

### ·Silane coupling material component

α-branched RO SC material (c1): 3 parts by mass of MPTiPS

### ·Polymerization initiator (F) component

2 parts by mass of PhBNa and 1 parts by mass of AcTU.

Note that the composition described above is expressed such that the total amount of the (A) component contained in the first agent and the second agent is 100 parts by mass. Further, the first agent is prepared by mixing the (a1), (a2), and (F), completely dissolving them, and then mixing the silica filler (B) therein, and the second agent is prepared by mixing the (b2), (C), and (F), completely dissolving them, and then mixing the (B) therein.

### (2) Preparation of dental adhesive composition

The first agent and the second agent prepared as described above are mixed at a mass ratio of 1:1 to prepare a dental adhesive composition to be evaluated.

### (3) Evaluation method for preparation of dental adhesive composition

The adhesive strength of the dental adhesive composition obtained in (2) was evaluated. In the evaluation, in order to examine the preservation stability of the first agent and the second agent, measurement was performed on two types of samples, i.e., (a) a sample that includes a dental adhesive composition prepared using the first agent and the second agent within three hours after preparation (sample "immediately after preparation") and (b) a sample that includes a dental adhesive composition prepared using the first agent and the second agent preserved in separate containers at 50°C for 2 weeks (sample "after preservation"), as samples. Further, the adhesive strength was measured as follows as the adhesive strength to porcelain that is silica ceramics.

First, porcelain was polished with #800 water-resistant abrasive paper while pouring water to scrape out the flat surface. After blowing compressed air onto this flat surface to dry it, a double sided tape having a circular hole with a diameter of 3 mm was fixed to define the adhesion area. Subsequently, the dental adhesive composition prepared by the method described above was applied to a columnar metal attachment having a diameter of 8 mm and the application surface of the dental adhesive composition was pressed against the porcelain surface such that the circular hole of the double sided tape and the surface of the metal attachment were concentric. After immersing this test sample in water at 37°C for 24 hours, a universal testing machine (AG-I type, manufactured by Shimadzu Corporation) was used to apply a load under the condition a crosshead speed of 1 mm/mm and apply a load to the test sample until the measurement sample is ruptured, thereby obtaining the adhesive strength using the following formula (2) on the basis of the maximum weight.

-Formula (2) Adhesive strength (MPa) = Maximum weight (N) / adhesion area(mm²).

### (4) Evaluation results

As a result of evaluation on the basis of the evaluation method described above, the adhesive strength to porcelain was 15.4 (MPa) immediately after preparation and 13.2 (MPa) after preservation. From this result, it has found that the dental adhesive composition according to Example 1 has excellent adhesiveness and preservation stability.

### Examples 2 to 35 and Comparative Examples 1 to 10

The first agent and the second agent having the compositions shown in Tables 3 to 5 were prepared in the same manner as that in Example 1. Note that also in these Tables, the notation method is the same as those in Tables 1 and 2. Further, for the parts by mass of the respective components of the first agent monomer composition and the second agent monomer composition used in the respective Examples and Comparative Examples in Tables 3 to 5, the parts by mass in Table 1 and Table 2 can be applied as they are, and the total amount of the (A) component contained in the first agent and the second agent is 100 parts by mass.

The preparation of the dental adhesive composition was evaluated in the same manner as that in Example 1 using the first agent and the second agent prepared in this way. The results are shown in Tables 3 to 5. Note that the "upward arrow" in the Tables means the same as above.

**[Table 3]**

| Example No. | Composition of first agent | | | Composition of second agent | | | Adhesive strength (MPa) | |
|---|---|---|---|---|---|---|---|---|
| | First agent monomer composition | Filler | Water (D) | Second agent monomer composition | Filler | SC material | | |
| | | Silica (B) | | | Silica (B) | Branched RO (C) | Immediately after preparation | After preservation |
| 1 | I -1 | F1(54) / F2(36) | - | II -1 | F1(54) / F2(36) | MPTiPS(3) | 15.4 | 13.2 |
| 2 | ↑ | ↑ | 0.3 | ↑ | ↑ | ↑ | 27.4 | 24.3 |
| 3 | ↑ | ↑ | ↑ | ↑ | ↑ | MPTiPS(0.1) | 13.9 | 13.1 |
| 4 | ↑ | ↑ | ↑ | ↑ | ↑ | MPTiPS(1) | 15.6 | 14.2 |
| 5 | ↑ | ↑ | ↑ | ↑ | ↑ | MPTiPS(2) | 18.3 | 16.5 |
| 6 | ↑ | ↑ | ↑ | ↑ | ↑ | MPTiPS(10) | 25.3 | 23.3 |
| 7 | ↑ | ↑ | ↑ | ↑ | ↑ | MPTiPS(15) | 19.6 | 18.2 |
| 8 | ↑ | ↑ | ↑ | ↑ | ↑ | MFFiPS(20) | 14.2 | 13.6 |
| 9 | I -2 | ↑ | ↑ | ↑ | ↑ | MPTiPS(3) | 13.2 | 12.1 |
| 10 | I -3 | ↑ | ↑ | ↑ | ↑ | ↑ | 19.4 | 18.1 |
| 11 | I -4 | ↑ | ↑ | ↑ | ↑ | ↑ | 24.4 | 22.3 |
| 12 | I -5 | ↑ | ↑ | ↑ | ↑ | ↑ | 18.7 | 17.9 |
| 13 | I -6 | ↑ | ↑ | ↑ | ↑ | ↑ | 14.6 | 13.1 |
| 14 | I -1 | F1(15) / F2(10) | ↑ | ↑ | F1(15) / F2(10) | ↑ | 16.3 | 15.9 |
| 15 | ↑ | F1(30) / F2(20) | ↑ | ↑ | F1(30) / F2(20) | ↑ | 24.5 | 21.5 |
| 16 | ↑ | F1(90) / F2(60) | ↑ | ↑ | F1(90) / F2(60) | ↑ | 23.4 | 21.3 |
| 17 | ↑ | F1(300) / F2(200) | ↑ | ↑ | F1(300) / F2(200) | ↑ | 14.2 | 12.5 |

**[Table 4]**

| Example No. | Composition of first agent | | | Composition of second agent | | | | | | Adhesive strength (MPa) | |
|---|---|---|---|---|---|---|---|---|---|---|---|
| | First agent monomer composition | Filler | Water (D) | Second agert monomer composition | Filler | | SC material | | Water (D) | | |
| | | Silica (B) | | | Silica (B) | Other (E) | Branched RO (C) | Other | | Immediately after preparation | After preservation |
| 18 | I -1 | F1(54) | 0.3 | II-1 | F1(54) | - | MPDiPMS(3) | - | - | 26.5 | 23.4 |
| | | F2(36) | | | F2(36) | | | | | | |
| 19 | ↑ | ↑ | ↑ | ↑ | ↑ | - | MPTtBS(3) | - | - | 24.4 | 19.3 |
| 20 | ↑ | ↑ | ↑ | ↑ | ↑ | - | MHTiPS(3) | - | - | 23.4 | 19.0 |
| 21 | ↑ | ↑ | 0.4 | ↑ | ↑ | - | MPTiPS(0.1) | - | - | 11.3 | 10.1 |
| 22 | ↑ | ↑ | 0.1 | ↑ | ↑ | - | ↑ | - | - | 16.6 | 14.3 |
| 23 | ↑ | ↑ | - | ↑ | ↑ | - | MPTiPS(1) | - | 0.5 | 17.3 | 13.9 |
| 24 | ↑ | ↑ | - | ↑ | ↑ | - | ↑ | - | 1 | 18.1 | 12.9 |
| 25 | ↑ | ↑ | - | ↑ | ↑ | - | ↑ | - | 1.5 | 16.1 | 10.2 |
| 26 | I-7 | ↑ | 0.3 | ↑ | ↑ | - | HPTiPS(3) | - | - | 27.2 | 23.1 |
| 27 | I-1 | ↑ | ↑ | ↑ | ↑ | G1(11) | ↑ | - | - | 22.3 | 20.5 |
| | | | | | | G2(7) | | | | | |
| 28 | ↑ | ↑ | ↑ | ↑ | ↑ | G1(22) | ↑ | - | - | 15.3 | 12.2 |
| | | | | | | G2(14) | | | | | |
| 29 | I-8 | ↑ | ↑ | II-2 | ↑ | - | ↑ | - | - | 27.6 | 23.8 |
| 30 | I-9 | ↑ | ↑ | II-3 | ↑ | - | ↑ | - | - | 24.3 | 22.2 |
| 31 | I-10 | ↑ | ↑ | II-4 | ↑ | - | ↑ | - | - | 26.3 | 22.5 |
| 32 | I-11 | ↑ | ↑ | II-5 | ↑ | - | ↑ | - | - | 25.1 | 21.9 |
| 33 | I-12 | ↑ | ↑ | II-6 | ↑ | - | ↑ | - | - | 27.6 | 23.7 |
| 34 | I-13 | ↑ | ↑ | II-1 | ↑ | - | ↑ | MPS(0.03) | - | 26.2 | 22.3 |
| 35 | ↑ | ↑ | ↑ | ↑ | ↑ | - | ↑ | MPS(0.09) | - | 25.3 | 15.5 |

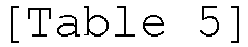

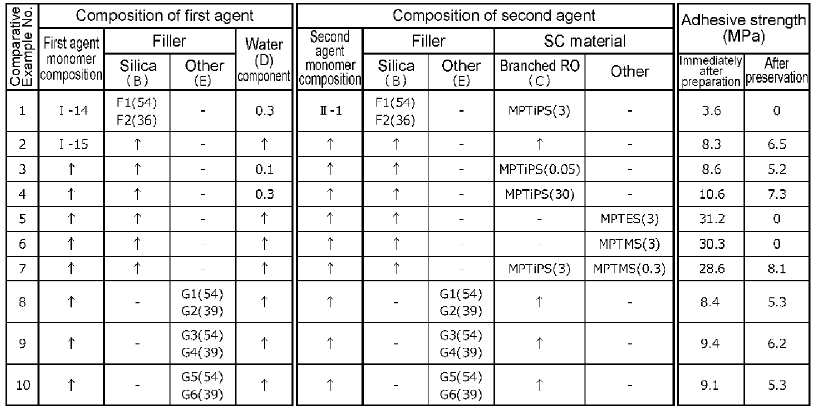

As shown in Table 3 and Table 4, in Examples 1 to 35 in which the components were mixed such that the configuration shown in the present invention is satisfied, the adhesive strength immediately after preparation and the adhesive strength after preservation were high, i.e., 10 MPa or more. In particular, in the case where the mixing amount of the branched RO SC material (c1) was 3 to 12 parts by mass with respect to 100 parts by mass of the monomer (A) and 1.8 to 12 parts by mass of the water (D) was mixed in the first agent with respect to 100 parts by mass of the branched RO SC material (c1), the adhesive strength immediately after preparation was high, i.e., 20 MPa, and adhesive strength equivalent to that immediately after preparation was exhibited even after preservation.

Meanwhile, in the Comparative Examples 1 to 10 in which the components were mixed such that the configuration shown in the present invention is not satisfied, favorable results were not obtained as shown in Table 5. That is, the Comparative Examples 1 to 4 are examples in which the content of the acidic group-containing polymerizable monomer or the SC material does not satisfy the configuration shown in the present invention, and exhibited low adhesive strength. The Comparative Examples 5 and 6 are examples in which only MPTES or MPTMS that is the SC material(c2) other than the branched RO SC material (c1) is included, and exhibited low adhesive strength after preservation. The Comparative Example 7 is an example in which the content ratio of the branched RO SC material (c1) in the SC material is the lower limit value or less, and exhibited low adhesive strength after preservation. The Comparative Examples 8 to 10 are examples in which the silica filler is not used, and exhibited low adhesive strength initially and after preservation.

## Claims

1. A dental adhesive composition, comprising:
100 parts by mass of a polymerizable monomer component (A) in which 1 to 30 parts by mass is an acidic group-containing polymerizable monomer (a1) and the remainder is an acidic group-free polymerizable monomer (a2);
50 to 1000 parts by mass of a silica filler (B); and
0.1 to 20 parts by mass of a silane coupling material (C), the dental adhesive composition being **characterized in that**
97 mass% or more of the silane coupling material (C) is an α-branched alkoxysilane silane coupling material (c1) formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position.

2. The dental adhesive composition according to claim 1, further comprising
1.5 to 300 parts by mass of water (D) with respect to 100 parts by mass of the silane coupling material (C).

3. The dental adhesive composition according to claim 1 or 2, further comprising
a polymerization initiator (E).

4. A two-paste type dental adhesive composition for preparing the dental adhesive composition according to any one of claims 1 to 3 by mixing a paste-like first agent and a paste-like second agent, comprising:
the first agent and the second agent, which are separately packaged, the two-paste type dental adhesive composition being **characterized in that**
the first agent contains the acidic group-containing polymerizable monomer (a1), the acidic group-free polymerizable monomer (a2), and the silica filler (B) and does not contain the silane coupling material (C),
the second agent contains the acidic group-free polymerizable monomer (a2), the silica filler (B), and the silane coupling material (C) and does not contain the acidic group-containing polymerizable monomer (a1), and
in a case where the dental adhesive composition contains components other than the acidic group-containing polymerizable monomer (a1), the acidic group-free polymerizable monomer (a2), the silica filler(B), and the silane coupling material (C), the components are contained in the first agent and/or the second agent.

5. The two-paste type dental adhesive composition according to claim 4, wherein
the first agent and/or the second agent contains 1.5 to 300 parts by mass of water (D) with respect to 100 parts by mass of the silane coupling material (C).

6. The two-paste type dental adhesive composition according to claim 5, wherein
3 to 12 parts by mass of the α-branched alkoxysilane silane coupling material (c1) is contained with respect to 100 parts by mass of the polymerizable monomer component (A), the first agent contains 1.8 to 12 parts by mass of water (D) with respect to 100 parts by mass of the α-branched alkoxysilane silane coupling material (c1), and the second agent does not contain water(D) or contains 10 parts by mass or less of water (D).

7. Dental cement, comprising:
the dental adhesive composition according to any one of claims 1 to 3.

8. Two-paste type dental cement, comprising:
the two-paste type dental adhesive composition according to any one of claims 4 to 6.

## Amended claims

### Amended claims under Art. 19.1 PCT

1. (Cancelled)

2. (Cancelled)

3. (Cancelled)

4. (Amended) A two-paste type dental adhesive composition for preparing a dental adhesive composition by mixing a paste-like first agent and a paste-like second agent, comprising:
the first agent and the second agent, which are separately packaged;
100 parts by mass of a polymerizable monomer component (A) in which 1 to 30 parts by mass is an acidic group-containing polymerizable monomer (a1) and the remainder is an acidic group-free polymerizable monomer (a2);
50 to 1000 parts by mass of a silica filler (B); and
0.1 to 20 parts by mass of a silane coupling material (C), the two-paste type dental adhesive composition being **characterized in that**
97 mass% or more of the silane coupling material (C) is an α-branched alkoxysilane silane coupling material (c1) formed of an organosilicon compound in which a monovalent radically polymerizable organic residue and a branched alkoxy group are bonded to a silicon atom, the monovalent radically polymerizable organic residue having at least one radically polymerizable functional group, the branched alkoxy group having 3 to 5 carbon atoms and a branch at an α position,
the first agent contains the acidic group-containing polymerizable monomer (a1), the acidic group-free polymerizable monomer (a2), and the silica filler (B) and does not contain the silane coupling material (C),
the second agent contains the acidic group-free polymerizable monomer (a2), the silica filler (B), and the silane coupling material (C) and does not contain the acidic group-containing polymerizable monomer (a1), and
in a case where the dental adhesive composition contains components other than the acidic group-containing polymerizable monomer (a1), the acidic group-free polymerizable monomer (a2), the silica filler(B), and the silane coupling material (C), the components are contained in the first agent and/or the second agent.

5. (Amended) The two-paste type dental adhesive composition according to claim 4, further comprising
1.5 to 300 parts by mass of water (D) with respect to 100 parts by mass of the silane coupling material (C), and
the water (D) is contained in the first agent and/or the second agent.

6. (Amended) The two-paste type dental adhesive composition according to claim 5, wherein
3 to 12 parts by mass of the α-branched alkoxysilane silane coupling material (c1) is contained in the two-paste type dental adhesive composition with respect to 100 parts by mass of the polymerizable monomer component (A), the first agent contains 1.8 to 12 parts by mass of water (D) with respect to 100 parts by mass of the α-branched alkoxysilane silane coupling material (c1), and the second agent does not contain water(D) or contains 10 parts by mass or less of water (D) .

7. (Cancelled)

8. Two-paste type dental cement, comprising:
the two-paste type dental adhesive composition according to any one of claims 4 to 6.
